# EUROPÄISCHE PATENTSCHRIFT

(11) **EP 0 343 534 B1**
(45) Veröffentlichungstag und Bekanntmachung des Hinweises auf die Patenterteilung: **29.11.1995**
(21) Anmeldenummer: 89109066.4
(22) Anmeldetag: 19.05.1989
(51) Int. Cl.: A61B 5/14, A61B 10/00, A61J 19/00, B65D 41/34

(54) **Flüssigkeitsaufbewahrungsbehälter insbesondere für Körperflüssigkeiten**
Container for preserving liquids, in particular bodily liquids
Récipient pour conserver des liquides, en particulier des liquides corporels

(30) Priorität: 27.05.1988 DE 3818115
(43) Veröffentlichungstag der Anmeldung: 29.11.1989
(73) Patentinhaber: Walter Sarstedt Geräte und Verbrauchsmaterial für Medizin und Wissenschaft, D-51588 Nümbrecht (DE)
(72) Erfinder: Vollmar, Herbert, Dipl.-Ing., D-5203 Much (DE)

(56) Entgegenhaltungen:
- EP-A- 0 137 352
- DE-A- 3 505 892
- FR-A- 2 206 253
- FR-A- 2 292 456
- GB-A- 2 096 584
- GB-A- 2 099 800
- US-A- 3 455 478
- US-A- 3 737 064
- US-A- 4 459 997
- US-A- 4 592 476
- US-A- 4 736 859
- US-A- 4 784 281

## Beschreibung

Die Erfindung betrifft einen Flüssigkeitsaufbewahrungsbehälter nach dem Oberbegriff des Patentanspruchs 1.

Derartige Flüssigkeitsaufbewahrungsbehälter werden z.B. zum Transport von durch einen Arzt oder eine Krankenschweste reinem Patienten abgenommene Körperflüssigkeiten zu einem Untersuchungslabor verwendet. Bei den in das Röhrchen eingefüllten Körperflüssigkeiten kann es sich um Urin, Speichel oder Blut handeln. Im allgemeinen weisen derartige Flüssigkeitsaufbewhrungsbehälter an ihrem offenen Ende einen abnehmbaren Verschluß auf, während sie am anderen Ende flüssigkeitsdicht verschlossen sind. Durch Öffnen des Verschlusses am vorderen Ende kann die Flüssigkeit in das Röhrchen eingefüllt werden, worauf dann der Verschluß angebracht und das Röhrchen zu einem Labor geschickt wird, wo der Verschluß entfernt und die Flüssigkeit vor oder nach einer Handhabung z.B. in einer Zentrifuge einer weiteren Nutzung zugeführtw ird.

Ein Problem bei derartigen Flüssigkeitsaufbewahrungsbehältern besteht darin, daß jede Manipulation in dem Sinne vermieden werden sollte, daß nach dem Verschließen des mit Flüssigkeit gefüllten Röhrchens durch die befugte Person bis zum Eintreffen des Flüssigkeitsaufnahmebehälters im Labor jedes erneute Öffnen des Behälters verhindert oder zumindest so gestaltet wird, daß es bei der Ankunft des Röhrchens im Labor erkannt wird. Auf diese Weise soll verhindert werden, daß Unbefugte entweder die in dem Röhrchen befindliche FLüssigkeit gegen eine andere austauschen oder irgendwelche anderen Substanzen in das Röhrchen zusätzlich eingeben.

Derartige Manipulationen müssen z.B. befürchtet werden, wenn sich in dem Röhrchen eine einem Kraftfahrer abgenommene Blutprobe befindet, die im Labor auf Alkoholgehalt untersucht werden soll.

Aus der FR-A-2 292 456 ist ein Flüssigkeitsaufbewahrungsbehälter mit einem einseitig offenen, zylindrischen Röhrchen bekannt, welches durch einen zum Einfüllen oder Entnehmen von Flüssigkeit abnehmbaren, am vorderen Ende des Röhrchens vorgesehene Verschluß flüssigkeitsdicht verschlossen ist, und mit einer am vorderen Ende des Röhrchens aufsetzbaren Verschlußkappe. Die Verschlußkappe und der Verschluß sind gemeinsam vom Behälter ab- und auch wieder auf diesen aufschraubbar, wodurch der Inhalt des Behälters manipuliert werden kann, ohne daß dies später erkennbar ist.

Aus der DE-A-3 505 892 ist ein verschließbarer Behälter zur sicheren Aufnahme von infektiösem Labormaterial bekannt, welcher aus einem Aufnahmegefäß und einem Deckel besteht, der über einen Schnappverschluß untrennbar mit dem Aufnahmegefäß verbunden werden kann. Der Behälter weist keine zusätzlich zum Deckel vorhandene Verschlußkappe auf.

Das Ziel der Erfindung besteht somit darin, einen Flüssigkeitsaufbewahrungsbehälter der eingangs genannten Gattung zu schaffen, an dem durch unbefugte Personen nach dem Einfüllen einer Flüssigkeit und dem Anbringen des Verschlusses nicht unerkennbar in der Weise manipuliert werden kann, daß der Inhalt des Behälters verfälscht werden kann, wobei ein dennoch vorgenommenes unbefugtes Öffnen am Bestimmungsort ohne weiteres erkannt werden soll.

Zur Lösung dieser Aufgabe sind die Merkmale des kennzeichnenden Teils des Patentanspruchs 1 vorgesehen.

Der Erfindungsgedanke ist also darin zu sehen, daß die befugte Person, welche das Röhrchen mit Flüssigkeit gefüllt und den Verschluß angebracht hat, die erfindungsgemäße Verschlußkappe von Hand, d.h. ohne Werkzeug auf das vordere Ende des verschlossenen Röhrchens aufschieben kann, wobei der Rastring so auf das am Röhrchen vorgesehene Rastmittel aufschnappt, dar ein Abnehmen der Verschlußkappe ohne Zerstörung der aufreißbaren Verbindung zwischen dem Rastring und der Verschlußkappe nicht möglich ist. Dadurch werden Personen, die den Behälter unbefugt öffnen wollen, gewarnt, dies zu tun, weil sie nicht in der Lage sind, die aufgebrochene Soll-Reiß-Verbindung zwischen der Verschlußkappe und dem Rastring wieder zu schließen. Sollte also eine unbefugte Person gleichwohl die Verschlußkappe unter Trennung von dem Rastring abreißen, so wird dies am Bestimmungsort, beispielsweise in dem Untersuchungslabor erkannt, und die in dem Röhrchen befindliche Flüssigkeit kann als für die Untersuchung ungeeignet verworfen werden. Es besteht dann die Möglichkeit, sofort beim Absender eine neue Flüssigkeitsprobe anzufordern.

Sogenannte Originalitätsverschlüsse für die verschiedensten Arten von Flüssigkeitsbehältern sind an sich bekannt; sie werden jedoch bereits beim Hersteller mittels geeigneter Maschinen und Werkzeuge schon bei der Herstellung des Verschlusses angebracht, so daß ein Füllen des Behälters außerhalb der Fabrik nicht möglich ist. Der Grundgedanke der vorliegenden Erfindung besteht demgegenüber darin, daß die den abreißbaren Rastring aufweisende Verschlußkappe im noch vom Flüssigkeitsaufbewahrungsbehälter getrennten Zustand an den Verbraucher geliefert wird, welcher dann die Verschlußkappe erst nach der Benutzung des Flüssigkeitsaufbewahrungsbehälters, d.h. nach dem Einfüllen der bestimmungsgemäßen Flüssigkeit von Hand und ohne besonderes Werkzeug die Verschlußkappe zum Versiegeln des Behälters aufbringen kann. Der an sich schon vorhandene Verschluß für das Röhrchen wird somit durch die zusätzlich noch angebrachte Verschlußkappe gegen unbefugtes Öffnen gesichert. Die Sollreißstellen müssen für die Handhabung und das Aufsetzen ausreichend stabil sein.

Bei dem Ausführungsbeispiel nach Anspruch 2 kann der Ringvorsprung bei der Herstellung des Röhrchens aus Kunststoff ohne weiteres einstückig mit angeformt werden. Der Ringvorsprung ist vorzugsweise koaxial zum kreiszylindrischen Röhrchen und weist eine gleichmäßige Breite und Stärke auf.

Die Ausbildung des Rastringes nach Anspruch 3 gewährleistet ein relativ zwangloses Aufschnappen auf das Rastmittel. Gleichwohl wird ein erheblicher Widerstand gegen axiales Abziehen herbeigeführt, so daß beim axialen Ziehen an der Verschlußkappe nicht der Rastring sich von dem Rastmittel löst, sondern vielmehr die Sollbruchstellen zwischen der Verschlußkappe und dem Rastring zerreißen.

Mit besonderem Vorteil wird die Erfindung bei einem Flüssigkeitsaufnahmebehälter mit einer das vordere Ende des Röhrchens übergreifenden Schraubkappe als Verschluß angewendet. Derartige Flüssigkeitsaufnahmebehälter werden häufig zur Blutentnahme verwendet (DE-PS 29 48 653).

Besonders vorteilhaft ist die Ausführungsform nach Anspruch 4, weil hierdurch die Manipulation mit Werkzeugen an dem Rastring bzw. dem Rastmittel vermieden wird, denn der Abdeckring verhindert das Angreifen mit Werkzeugen an der Rastvorrichtung. Sollte der Abdeckring mittels Werkzeugen zerstört werden, so ist dies im Labor erkennbar ebenso wie das Trennen der Verschlußkappe vom Rastring.

Die Erfindung ist auch mit besonderem Vorteil bei Flüssigkeitsaufnahmebehältern anwendbar, die einen vom Verschluß nach vorn vorstehenden, vorzugsweise zylindrischen, einen axialen Durchgangskanal aufweisenden Ansatz aufweisen, in dem ein vom rückwärtigen Ende einer beidseits angeschärften Kanüle durchstechbares Verschlußglied angeordnet ist (DE-PS 29 48 653). In diesem Fall können zweckmäßigerweise noch die Maßnahmen nach Anspruch 5 vorgesehen sein, welche etwa im Bereich des Verschlußgliedes austretendes Blut am weiteren Auslaufen hindern.

Die auf das Röhrchen aufgebrachte Schraubkappe weist im allgemeinen eine aus axialen Rippen bestehende Oberflächenprofilierung auf. In diesem Fall sieht die Erfindung nach Anspruch 6 vor, daß zwischen der aufgesetzten Verschlußkappe und der Schraubkappe eine drehfeste Verbindung vorliegt, die es gestattet, durch Drehen der Verschlußkappe die Verschlußkappe abzuschrauben. Die Verschlußkappe dient hierbei also zusätzlich noch als Werkzeug zur Betätigung der Schraubkappe.

Die Anordnung der Verschlußkappe zum Verschluß ist in Anspruch 7 gekennzeichnet.

In Anspruch 8 ist ein vorteilhaftes Verfahren zum Sichern von Flüssigkeitsaufbewahrungsbehältern gemäß der Erfindung gekennzeichnet.

Die Erfindung wird im folgenden beispielsweise anhand der Zeichnung beschrieben; in dieser zeigt:
- Fig. 1: eine schematische Seitenansicht eines erfindungsgemäßen Flüssigkeitsaufbewahrungsbehälters in Form einer Blutabnahmevorrichtung in Gebrauchsstellung noch ohne aufgesetzte Verschlußkappe,
- Fig. 2: eine vergrößerte Schnittansicht des vorderen Teils des Flüssigkeitsaufbewahrungsbehälters nach Fig. 1,
- Fig. 3: eine Schnittansicht analog Fig. 2, wobei jedoch die Führungshülse mit der Kanüle abgenommen und stattdessen eine erfindungsgemäße Verschlußkappe mit Rastring auf das vordere Ende des Röhrchens aufgesetzt ist,
- Fig. 4: eine schematische perspektivische Ansicht der erfindungsgemäßen Verschlußkappe mit Rastring,
- Fig. 5: einen schematischen Schnitt nach Linie V-V in Fig. 3,
- Fig. 6: einen Schnitt nach Linie VI-VI in Fig. 3, und
- Fig. 7: einen Schnitt nach Linie VII-VII in Fig. 3.

In allen Figuren der Zeichnung bezeichnen gleiche Bezugszahlen einander entsprechende Bauteile.

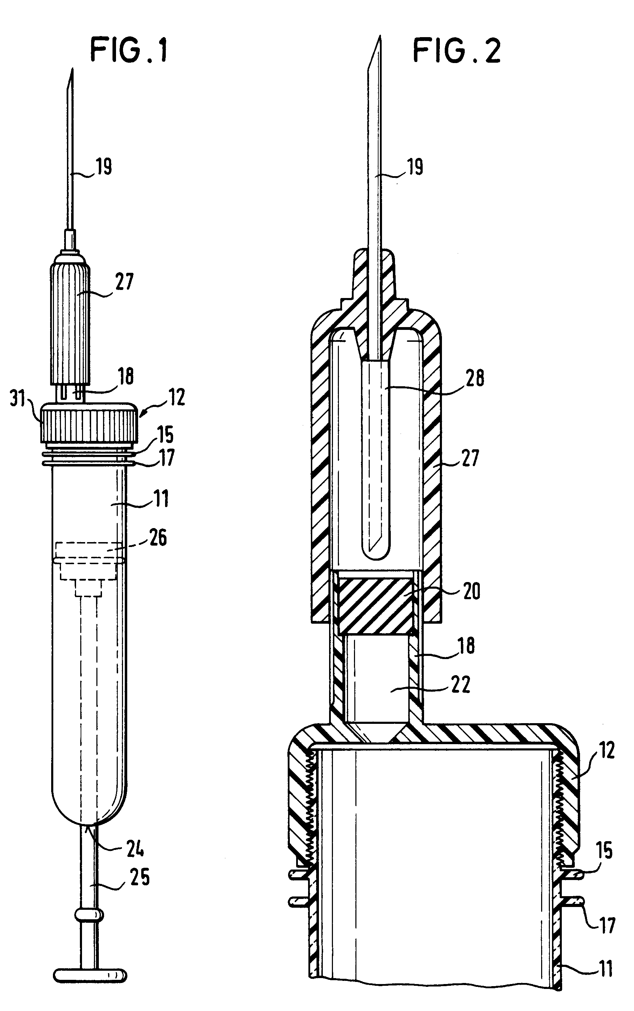

Nach den Fig. 1 und 2 erstreckt sich durch das abgerundete und mit einer Durchtrittsöffnung versehene hintere Ende 24 eines aus Kunststoff bestehenden kreiszylindrischen Röhrchens 11 gleitend eine Kolbenstange 25, die an ihrem vorderen Ende einen axial gleitend im Innern des Röhrchens 11 angeordneten Kolben 26 trägt. Auf das mit einem Außengewinde versehene vordere Ende des Röhrchens 11 ist eine Schraubkappe 12 aufgeschraubt, welche einen axial nach vorn vorstehenden kreiszylindrischen Ansatz 18 einstückig trägt, in dem ein axialer Durchgangskanal 22 vorgesehen ist, der wiederum an seinem vorderen Ende durch ein elastisches Verschlußglied 20 verschlossen ist.

Auf den Ansatz 18 ist von vorn eine Führungshülse 27 aufgeschoben, die eine kreiszylindrische und zum Ansatz 18 komplementäre Innenfläche aufweist. Die Führungshülse 27 ist an ihrem rückwärtigen Ende offen und am vorderen Ende verschlossen. Dort wird sie von einer an ihr befestigten Kanüle 19 durchgriffen, die an beiden Enden angeschärft ist. Das rückwärtige Ende ist von einem elastischen Schlauch 28 überzogen.

Wird die Führungshülse 27 aus der Position nach Fig. 2 weiter auf den Ansatz 18 aufgeschoben, so durchbohrt das rückwärtige Ende der Kanüle 19 zunächst den elastischen Schlauch 28 und dann den Verschlußstopfen 20, den sie schließlich vollständig durchdringt, um in den Durchgangskanal 22 zu münden. Der Schlauch 28 schiebt sich dabei ziehharmonikaartig zusammen. Nunmehr kann mit der gezeigten Anordnung Blut aus der Vene eines Patienten entnommen werden, in die die Kanüle 19 eingestochen ist. Hierbei wird der Kolben 26 zurückgezogen.

Soweit, wie bisher beschrieben, ist der zur Blutentnahme dienende Flüssigkeitsaufnahmebehälter aus der DE-PS 29 48 653 bekannt.

Erfindungsgemäß ist nach Fig. 2 an der Außenwand des Röhrchens 11 unmittelbar hinter der aufgeschraubten Schraubkappe 12 ein einstückiger Ringvorsprung 15 vorgesehen, der koaxial mit dem Röhrchen 11 ist. In geringem Abstand dahinter befindet sich ein ebenfalls zum Röhrchen 11 koaxialer Abdeckring 17.

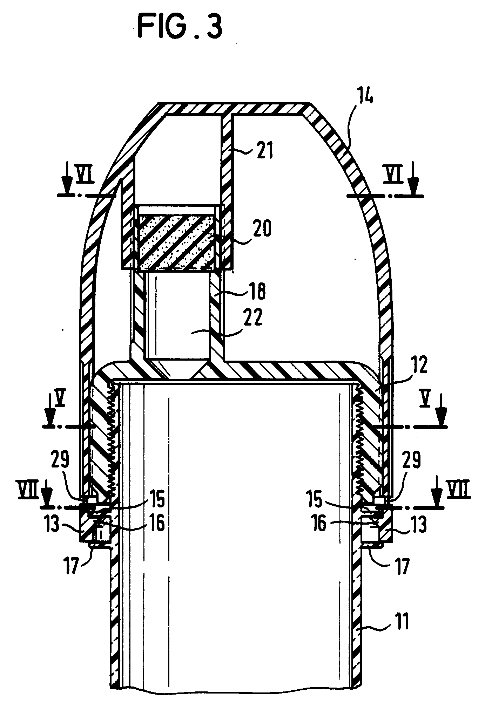

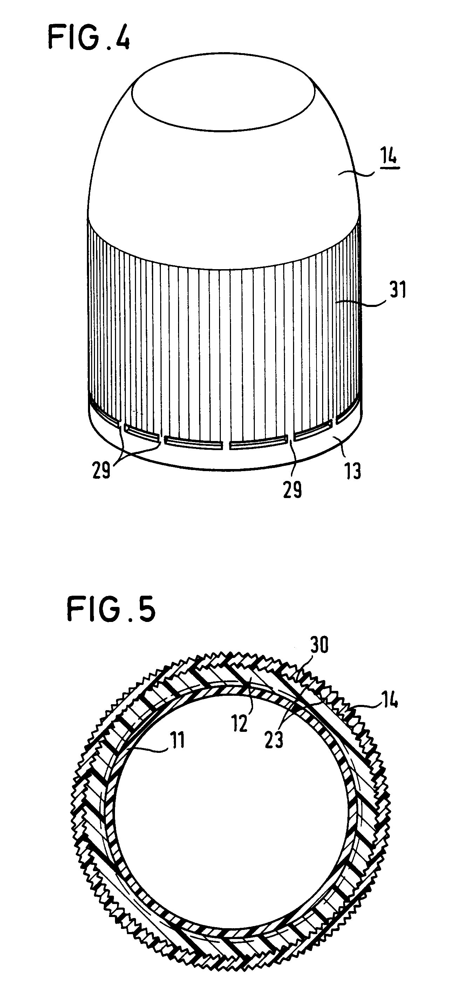

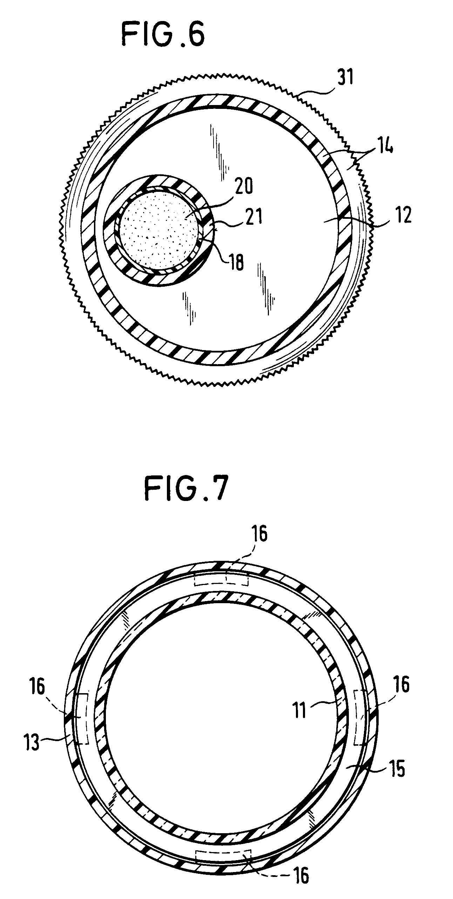

Nach der Blutentnahme wird die Führungshülse 27 nach vorn abgezogen, worauf eine in Fig. 4 dargestellte Verschlußkappe 14 mit einem an ihrem offenen Ende über Sollbruchstellen 29 angeordneten Rastring 13 gemäß Fig. 3 von vorn auf das mit der Schraubkappe 12 versehene Röhrchen 11 aufgeschoben wird. Der Rastring 13 weist gemäß den Fig. 3 und 7 radial innen über den Umfang verteilt vier Rastvorsprünge 16 auf, die hinten zum Auflaufen auf den Ringvorsprung 15 abgeschrägt, vorn dagegen flach ausgebildet sind, damit sie nach dem zwanglosen Aufschnappen auf den Ringvorsprung 15 axial nicht mehr nach vorn über den Ringvorsprung 15 abgezogen werden können.

Der Innenumfang des Rastringes 13 ist so ausgebildet, daß er von vorn axial über den Ringvorsprung 15 paßt, so daß die schrägen Rückenflächen der Rastvorsprünge 16 auf den Ringvorsprung 15 aufgleiten und unter federnder Erweiterung des Rastringes 13 schließlich hinter den Ringvorsprung 15 schnappen können.

Der Innendurchmesser des rückwärtigen Endes der Verschlußkappe entspricht dem Außendurchmesser der Schraubkappe 12, so daß der rückwärtige Teil der Verschlußkappe 14 formschlüssig auf dem Außenumfang der Schraubkappe 12 aufliegt, welche nach den Fig. 4 und 5 außen mit einer Riffelung versehen ist, die aus axialen Rippen 23 besteht. Innen ist die Verschlußkappe 14 mit einer komplementären Oberflächenprofilierung 30 versehen, so daß bei gemäß Fig. 3 aufgesetzter Verschlußkappe 14 zwischen dieser und der Schraubkappe 12 eine drehfeste formschlüssige Verbindung vorliegt. Vorzugsweise verjüngt sich die Verschlußkappe 14 oberhalb der Schraubkappe 12 derart, daß ein wesentlich weiteres axiales Aufschieben der Verschlußkappe 14 auf die Schraubkappe 12, als es in Fig. 3 dargestellt ist, nicht möglich ist.

Der hintere Rand der Verschlußkappe 14 schließt vorzugsweise in etwa bündig mit dem hinteren Rand der Schraubkappe 12 ab. Der Rastring 13 befindet sich unmittelbar hinter der Schraubkappe 12.

Die axiale Ausdehnung des Rastringes 13 ist gemäß Fig. 3 derart, daß er sich bei aufgesetzter Verschlußkappe 14 nur etwa bis zum Abdeckring 17 erstreckt. Der Außendurchmesser des Abdeckringes 17 ist geringfügig größer als der Innendurchmesser des Rastringes 13 am hinteren Ende, so daß nach dem in Fig. 3 dargestellten Aufschnappen des Rastringes 13 auf den Ringvorsprung 15 eine weitere axiale Verschiebung des Rastringes 13 und damit der Verschlußkappe 14 nach hinten nicht möglich ist. Auf diese Weise ist die Position der Verschlußkappe 14 auf dem Röhrchen 11 eindeutig definiert.

An der vorderen Innenseite weist die Verschlußkappe 14 nach den Fig. 3 und 6 einen sich von der Vorderwand nach hinten axial erstreckenden Kappenvorsprung 21 auf, der einstückig an die vorzugsweise aus Kunststoff bestehende Verschlußkappe 14 mit angeformt ist und koaxial zum Ansatz 18 verläuft. Bei aufgesetzter Verschlußkappe 14 setzt sich der vorzugsweise kreiszylindrisch ausgebildete hohle Kappenvorsprung 21 in der aus den Fig. 3 und 6 ersichtlichen Weise axial auf den Ansatz 18 und verschließt diesen damit gegen ein unkontrolliertes Auslaufen von Blut, welches an der Vorderfläche des elastischen Verschlußgliedes 20 vorhanden sein könnte.

Erfindungsgemäß ist die Verschlußkappe auch auf ihrem. Außenumfang mit einer Riffelung 31 versehen, wie das in den Fig. 4 und 6 angedeutet ist.

Die Arbeitsweise des beschriebenen Flüssigkeitsaufnahmebehälters ist wie folgt:
Nach einer Blutentnahme wird die Führungshülse 27 (Fig. 2) abgezogen und entweder in der Vene des Patienten gelassen oder weggeworfen.

Auf das vordere Ende des von der Führungshülse 27 entblößten Flüssigkeitsbehälters wird dann gemäß Fig. 3 die aus Fig. 4 ersichtliche Verschlußkappe von vorn aufgeschoben, wobei die Rastvorsprünge 16 unter geringfügiger federnder Erweiterung des hinteren zylindrischen Teils der Verschlußkappe 14 am Außenumfang der Schraubkappe 12 entlanggleiten, bis sie schließlich hinter den Ringvorsprung 15 schnappen. Dabei setzt sich der Kappenvorsprung 21 dichtend oder zumindest das unkontrollierte Auslaufen von Blut verhindernd auf den Ansatz 18. In der aus Fig. 3 ersichtlichen Position kann der Flüssigkeitsaufnahmebehälter nunmehr zum Versand gebracht werden.

In dem Labor, wo der Inhalt des Röhrchens 11 untersucht werden soll, wird nunmehr die Schraubkappe 12 dadurch abgeschraubt, daß die Verschlußkappe 14 in Höhe der Schraubkappe 12 mit zwei Fingern ergriffen und entgegen dem Uhrzeigersinn gedreht wird. Hierbei wird das auf die Verschlußkappe 14 ausgeübte Drehmoment auf die Schraubkappe 12 übertragen und diese axial vom Röhrchen 11 entfernt. Da die Rastvorsprünge 16 axial unlösbar hinter den Ringvorsprung 15 greifen, brechen bei dem Abschraubvorgang die Sollbruchstellen 29 auf, und die Verschlußkappe 14 wird vom Rastring 13 getrennt, welcher aufgrund der beschriebenen baulichen Ausbildung zwischen dem Ringvorsprung 15 und dem Abdeckring 17 festgehalten bleibt.

Nach dem Abnehmen der Verschlußkappe 14 mit der Schraubkappe 12 kann der Inhalt des Röhrchens 11 entnommen werden.

Sollte die Schraubkappe 12 zwischenzeitlich entfernt und wieder aufgesetzt worden sein, so erkennt man dies ohne weiteres an der erfolgten Zerstörung der Sollbruchstellen 29.

Eine Abnahme der Verschlußkappe 14 ist auch nicht dadurch möglich, daß von hinten ein Werkzeug hinter den Rastring 13 geführt wird, weil dies durch den mit dem Röhrchen 11 einstückigen Abdeckring 17 verhindert wird.

Erfindungsgemäß kann der Kappenvorsprung 21 auch so ausgebildet werden, daß er auf einen üblichen Kanülenkonus an der Vorderseite der Schraubkappe 12 dichtend aufgesetzt werden kann. Hierzu wäre der Kappenvorsprung 21 komplementär zur Außenform des Kanülenansatzes auszubilden.

## Patentansprüche

1. Flüssigkeitsaufbewahrungsbehälter, insbesondere für Körperflüssigkeiten und / oder für den Transport von Flüssigkeiten von einer Füllstelle zu einer Untersuchungsstelle, mit einem einseitig offenenen, vorzugsweise zylindrischen Röhrchen (11), welches durch einen zum Einfüllen oder Entnehmen von Flüssigkeit abnehmbaren, am vorderen Ende des Röhrchens vorgesehenen Verschluß (12) flüssigkeitsdicht verschlossen ist, und mit einer am vorderen Ende des Röhrchens aufsetzbaren Verschlußkappe (14), dadurch gekennzeichnet,
daß hinter dem aufgesetzten Verschluß (12) an der Außenwand des Röhrchens (11) ein Rastmittel (15) vorgesehen und die Verschlußkappe (14) an ihrer offenen Seite mit einem abreißbaren Rastring (13) versehen und derart auf das vordere Ende des Röhrchens (11) aufsetzbar ist, daß der Rastring (13) über das Rastmittel (15) schnappt, worauf die Verschlußkappe (14) den Verschluß (12) nach außen abdeckt, derart, daß ein Öffnen des Verschlusses (12) ohne ein Abtrennen des Rastrings (13) von der Verschlußkappe (14) unmöglich ist.

2. Behälter nach Anspruch 1, dadurch gekennzeichnet,
daß das Rastmittel aus einem rund um das Röhrchen (11) vorgesehenen Ringvorsprung (15) besteht, der vorzugsweise einstückig mit dem Röhrchen (11) ist.

3. Behälter nach Anspruch 1 oder 2, dadurch gekennzeichnet,
daß der Rastring (13) radial innen über den Umfang verteilt Rastvorsprünge (16) trägt, die mit dem Rastmittel (15) in rastenden Eingriff treten.

4. Behälter nach einem der vorhergehenden Ansprüche,
dadurch gekennzeichnet,
daß in einem Abstand hinter dem Rastmittel (15) ein Abdeckring (17) an der Außenwand des Röhrchens (11) vorgesehen ist, der bei aufgesetzter Verschlußkappe (14) und unabgerissenem Rastring (13) den Rastring (13) und das Rastmittel (15) gegen Manipulation von außen abdeckt.

5. Behälter nach einem der vorhergehenden Ansprüche,
dadurch gekennzeichnet,
daß ein vom Verschluß (12) nach vorn vorstehender, vorzugsweise zylindrischer, einen axialen Durchgangskanal (22) aufweisender Ansatz (18) vorgesehen ist, in dem ein vom rückwärtigen Ende einer beidseitig angeschärften Kanüle (19) durchstechbares Verschlußglied (20) angeordnet ist und daß die Verschlußkappe (14) innen einen Kappenvorsprung (21) aufweist, der bei aufgesetzter Verschlußkappe (14) das vordere Ende des Ansatzes (18) abdeckt und dicht verschließt.

6. Behälter nach einem der Ansprüche 4 oder 5,
dadurch gekennzeichnet,
daß am Außenumfang der Schraubkappe (12) eine Oberflächenprofilierung (23) vorgesehen ist und die Verschlußkappe (14) auf ihrem im Durchmesser dem Außendurchmesser der Schraubkappe (12) entsprechenden Innenumfang eine komplementäre Oberflächenprofilierung (30) derart aufweist, daß die aufgesetzte Verschlußkappe (14) zumindest kraftschlüssig, vorzugsweise jedoch formschlüssig drehfest mit der Schraubkappe (12) verbunden ist.

7. Kombination eines Verschlusses (12) und einer Verschlußkappe (14) für einen Flüssigkeitsaufbewahrungsbehälter, insbesondere für Körperflüssigkeiten und / oder den Transport von Flüssigkeiten von einer Füllstelle zu einer Untersuchungsstelle, mit einem einseitig offenen, vorzugsweise zylindrischen Röhrchen (11), welches durch den zum Einfüllen oder Entnehmen von Flüssigkeit abnehmbaren, am vorderen Ende des Röhrchens (11) vorgesehenen Verschluß (12) flüssigkeitsdicht verschlossen ist und mit einer am vorderen Ende des Röhrchens aufgesetzten Verschlußkappe (14), dadurch gekennzeichnet,
daß die Verschlußkappe (14) an ihrer offenen Seite mit einem abreißbaren Rastring (13) versehen ist und in einer den Verschluß (12) nach außen abdeckenden Weise auf diesem derart angeordnet ist, daß der abreißbare Rastring (13) der Verschlußkappe (14) sich unmittelbar hinter dem hinteren Rand des Verschlusses (12) befindet.

8. Verfahren zum Sichern von Flüssigkeitsaufbewahrungsbehältern gegen unbefugtes Öffnen,
dadurch gekennzeichnet, daß ein Behälter nach einem der Ansprüche 1 bis 6 bereitgestellt, das Röhrchen (11) mit einer aufzubewahrenden und / oder zu transportierenden Flüssigkeit gefüllt und die Verschlußkappe (14) mit dem Rastring (13) von Hand axial auf das verschlossene vordere Ende des Röhrchens (11) aufgeschnappt wird.

## Claims

1. Fluid-storing container, especially for body fluids and/or for transporting fluids from a filling site to an examination site, having a preferably cylindrical tube (11) which is open at one end and is closed in a fluid-tight manner by a closure element (12) which can be removed for introducing or removing fluid and is provided at the front end of the tube, and having a closure cap (14) which can be placed on the front end of the tube, characterised in that a locking means (15) is provided behind the fitted closure element (12) on the outer wall of the tube (11) and the closure cap (14) is provided with a tear-off locking ring (13) at its open end and can be placed on the front end of the tube (11) in such a manner that the locking ring (13) snaps over the locking means (15), whereupon the closure cap (14) covers the closure element (12) from the outside in such a manner that it is not possible to open the closure element (12) without separating the locking ring (13) from the closure cap (14).

2. Container according to claim 1, characterised in that the locking means comprises an annular projection (15) which is provided around the tube (11) and is preferably integral with the tube (11).

3. Container according to claim 1 or 2, characterized in that the locking ring (13) carries locking protuberances (16) which are distributed radially on the inside over the circumference and which are in locking engagement with the locking means (15).

4. Container according to any one of the preceding claims, characterized in that a cover ring (17) is provided on the outer wall of the tube (11) at a distance behind the locking means (15) and covers the locking ring (13) and the locking means (15) against manipulation from outside when the closure cap (14) has been fitted and the locking ring (13) has not been torn off.

5. Container according to any one of the preceding claims, characterized in that a preferably cylindrical attachment (18) which projects forwards from the closure element (12) and has an axial through channel (22) is provided which accommodates a closure member (20) which can be perforated by the rear end of a cannula (19) which is sharpened at both ends and in that the closure cap (14) has, on the inside, a cap projection (21) which covers the front end of the attachment (18) and closes it in a sealing manner when the closure cap (14) has been fitted.

6. Container according to either of claims 4 and 5, characterised in that a surface profiling (23) is provided on the outer circumference of the screw cap (12) and the closure cap (14) has a complementary surface profiling (30) on its inner circumference which corresponds in diameter to the outside diameter of the screw cap (12), in such a manner that the fitted closure cap (14) is connected to the screw cap (12) in a rotationally secure manner and at least in non-positively locking manner but preferably in positively locking manner.

7. Combination of a closure element (12) and a closure cap (14) for a fluid-storing container, especially for body fluids and/or for transporting fluids from a filling site to an examination site, having a preferably cylindrical tube (11) which is open at one end and is closed in a fluid-tight manner by the closure element (12) which can be removed for introducing and removing fluid and is provided at the front end of the tube (11), and having a closure cap (14) fitted on the front end of the tube, characterised in that the closure cap (14) is provided with a tear-off locking ring (13) at its open end and is so arranged on the closure element (12) in a manner covering that closure element from the outside that the tear-off locking ring (13) of the closure cap (14) is located immediately behind the rear edge of the closure element (12).

8. Method of securing fluid-storing containers against unauthorised opening, characterised in that a container according to any one of claims 1 to 6 is provided, the tube (11) is filled with a fluid that is to be stored and/or transported and the closure cap (14) with the locking ring (13) is snapped axially onto the closed front end of the tube (11) by hand.

## Revendications

1. Récipient de conservation de liquide, en particulier pour des liquides corporels et/ou pour transporter des liquides d'un lieu de remplissage à un lieu d'analyse, comprenant une éprouvette (11), de préférence cylindrique, ouverte d'un côté et obturée de manière étanche aux liquides par un bouchon (12) qui se trouve à l'extrémité avant de l'éprouvette et qui peut être retiré pour verser ou prélever du liquide, et comprenant une coiffe (14) pouvant être mise en place à l'extrémité avant de l'éprouvette, caractérisé en ce qu'un moyen d'encliquetage (15) est prévu sur la paroi extérieure de l'éprouvette (11) derrière le bouchon (12) lorsque celui-ci est en place, et la coiffe (14) est munie, sur son côté ouvert, d'un anneau d'encliquetage arrachable (13) et peut être mise en place sur l'extrémité avant de l'éprouvette (11) par le fait que l'anneau d'encliquetage (13) s'emboîte par-dessus le moyen d'encliquetage (15), après quoi la coiffe (14) recouvre le bouchon (12) vers l'extérieur, de façon à rendre l'ouverture du bouchon (12) impossible sans séparer l'anneau d'encliquetage (13) de la coiffe (14).

2. Récipient selon la revendication 1, caractérisé en ce que le moyen d'encliquetage est constitué par une saillie annulaire (15) qui entoure l'éprouvette (11) et qui, de préférence, fait corps avec l'éprouvette (11).

3. Récipient selon la revendication 1 ou 2, caractérisé en ce que l'anneau d'encliquetage (13) comporte des saillies d'encliquetage (16) qui sont réparties radialement vers l'intérieur sur la périphérie et qui entrent en prise d'encliquetage avec le moyen d'encliquetage (15).

4. Récipient selon l'une des revendications précédentes, caractérisé en ce qu'à distance derrière le moyen d'encliquetage (15) est prévu, sur la paroi extérieure de l'éprouvette (11), un anneau de recouvrement (17) qui, lorsque la coiffe (14) est en place et l'anneau d'encliquetage (13) est intact, protège l'anneau d'encliquetage (13) et le moyen d'encliquetage (15) contre toute manipulation.

5. Récipient selon l'une des revendications précédentes, caractérisé en ce qu'il est prévu un embout (18), de préférence cylindrique, qui fait saillie vers l'avant sur le bouchon (12) et comporte un canal axial débouchant (22) et dans lequel est disposé un élément d'obturation (20) susceptible d'être percé par l'extrémité arrière d'une canule (19) affûtée des deux côtés, et en ce que la coiffe (14) comporte, à l'intérieur, un tube en saillie (21) qui, lorsque la coiffe (14) est en place, recouvre et obture de manière étanche l'extrémité avant de l'embout (18).

6. Récipient selon l'une des revendications 4 et 5, caractérisé en ce que la coiffe vissable (12) comporte un profilage superficiel (23) sur sa périphérie extérieure, et la coiffe (14) comporte un profilage superficiel complémentaire (30) sur sa périphérie intérieure dont le diamètre correspond au diamètre extérieur de la coiffe vissable (12), de façon que, une fois en place, la coiffe (14) soit solidarisée sans possibilité de rotation avec la coiffe vissable (12) au moins par force, mais de préférence par complémentarité de formes.

7. Combinaison d'un bouchon (12) et d'une coiffe (14) destinés à un récipient de conservation de liquide, en particulier pour des liquides corporels et/ou pour transporter des liquides d'un lieu de remplissage à un lieu d'analyse, comprenant une éprouvette (11), de préférence cylindrique, ouverte d'un côté et obturée de manière étanche aux liquides par le bouchon (12) qui se trouve à l'extrémité avant de l'éprouvette (11) et qui peut être retiré pour verser ou prélever du liquide, et comprenant une coiffe (14) pouvant être mise en place à l'extrémité avant de l'éprouvette, caractérisée en ce que la coiffe (14) est munie, sur son côté ouvert, d'un anneau d'encliquetage arrachable (13) et est disposée sur le bouchon (12) en le recouvrant vers l'extérieur, de façon que l'anneau d'encliquetage arrachable (13) de la coiffe (14) se trouve juste derrière le bord arrière du bouchon (12).

8. Procédé pour protéger des récipients de conservation de liquide contre une ouverture par une personne non habilitée, caractérisé en ce qu'il est prévu un récipient conforme à l'une des revendications 1 à 6, dont l'éprouvette (11) est remplie avec un liquide que l'on veut conserver et/ou transporter, et la coiffe (14) munie de l'anneau d'encliquetage (13) est encliquetée axialement à la main sur l'extrémité avant fermée de l'éprouvette (11).
